# EUROPÄISCHE PATENTSCHRIFT

(11) **EP 1 919 943 B1**
(45) Veröffentlichungstag und Bekanntmachung des Hinweises auf die Patenterteilung: **24.03.2010**
(21) Anmeldenummer: 06791770.8
(22) Anmeldetag: 31.08.2006
(51) Int. Cl.: C07K 14/47, C07K 16/18, C07K 19/00, A61K 38/17, C12N 15/12, C12N 15/62, C12N 15/63

(54) **MELANOM-ASSOZIIERTE MHC KLASSE I ASSOZIIERTE OLIGOPEPTIDE UND DEREN VERWENDUNGEN**
MELANOMA-ASSOCIATED MHC CLASS I ASSOCIATED OLIGOPEPTIDES AND THE USES THEREOF
OLIGOPEPTIDES ASSOCIES AU COMPLEXE D'HISTOCOMPATIBILITE PRINCIPALE (MHC) DE CLASSE I, ASSOCIES AU MELANOME ET LEURS UTILISATIONS

(30) Priorität: 01.09.2005 DE 102005041616
(43) Veröffentlichungstag der Anmeldung: 14.05.2008
(73) Patentinhaber: Johannes Gutenberg-Universität Mainz, 55122 Mainz (DE)
(72) Erfinder: EBERTS, Daniela, 55101 Mainz (DE); FATHO, Martina, 55286 Wörrstadt (DE); LENNERZ, Volker, 55270 Ober-Olm (DE); SCHMIDT, Chris, Herston, QLD 4006 (AU); VAN DER BRUGGEN, Pierre, B-1200 Brussels (BE); WÖLFEL, Catherine, 55128 Mainz (DE); WÖLFEL, Thomas, 55128 Mainz (DE)
(74) Vertreter: Krauss, Jan
(86) Internationale Anmeldenummer: PCT/EP2006/008533
(87) Internationale Veröffentlichungsnummer: WO 2007/025760

(56) Entgegenhaltungen:
- WO-A-01/11040
- WO-A1-01/90197
- DATABASE UniProt MAGEA4 protein 5. Juli 2005 (2005-07-05), XP002408519 accession no. Q4V9T5
- DATABASE UniProt Voltage-gated sodium channel NAV1.9 (Fragment) 1. Juni 2003 (2003-06-01), XP002408520 accession no. Q86XP4 -& DIB-HAJJ S D ET AL: "NaN, a novel voltage-gated Na channel, is expressed preferentially in peripheral sensory neurons and down-regulated after axotomy" PROCEEDINGS OF THE NATIONAL ACADEMY OF SCIENCES OF USA, NATIONAL ACADEMY OF SCIENCE, WASHINGTON, DC, US, Bd. 95, Juli 1998 (1998-07), Seiten 8963-8968, XP002110597 ISSN: 0027-8424
- REYNOLDS S R ET AL: "Identification of HLA-A*03, A*11 and B*07-restricted melanoma-associated peptides that are immunogenic in vivo by vaccine-induced immune response (VIIR) analysis" JOURNAL OF IMMUNOLOGICAL METHODS, ELSEVIER SCIENCE PUBLISHERS B.V.,AMSTERDAM, NL, Bd. 244, Nr. 1-2, 20. Oktober 2000 (2000-10-20), Seiten 59-67, XP004218446 ISSN: 0022-1759
- HERR W ET AL: "Detection and quantification of blood-derived CD8<+> T lymphocytes secreting tumor necrosis factor alpha in response to HLA-A2.1-binding melanoma and viral peptide antigens" JOURNAL OF IMMUNOLOGICAL METHODS, ELSEVIER SCIENCE PUBLISHERS B.V.,AMSTERDAM, NL, Bd. 191, Nr. 2, 1996, Seiten 131-142, XP004020840 ISSN: 0022-1759 in der Anmeldung erwähnt
- LENNERZ V ET AL.: "The response of autologous T cells to a human melanoma is dominated by mutated neoantigens" PROCEEDINGS OF THE NATIONAL ACADEMY OF SCIENCE USA (PNAS), Bd. 102, Nr. 44, 1. November 2005 (2005-11-01), Seiten 16013-16018, XP002408502

### SEQUENCE LISTING

<110> Johannes Gutenberg-Universität Mainz
<120> Melanom-assoziierte MHC Klasse I assoziierte Oligopeptide und deren Verwendungen
<130> U30122PCT
<160> 13
<170> PatentIn version 3.3
<210> 1
   <211> 9
   <212> PRT
   <213> Homo sapiens
<400> 1
   Trp Gln Tyr Phe Phe Pro Val Ile Phe
   1               5
<210> 2
   <211> 10
   <212> PRT
   <213> Homo sapiens
<400> 2
   Lys Arg Cys Phe Pro Val Ile Phe Gly Lys
   1 5 10
<210> 3
   <211> 9
   <212> PRT
   <213> Homo sapiens
<400> 3
   Lys Val Asp Glu Leu Ala His Phe Leu
   1 5
<210> 4
   <211> 9
   <212> PRT
   <213> Homo sapiens
<400> 4
   Asn Met Val Pro Phe Phe Pro Pro Val
   1 5
<210> 5
   <211> 10
   <212> PRT
   <213> Homo sapiens
<400> 5
   Met Pro Arg Glu Asp Ala His Phe Ile Tyr
   1 5 10
<210> 6
   <211> 10
   <212> PRT
   <213> Homo sapiens
<400> 6
   Leu Tyr Pro Glu Trp Thr Glu Ala Gln Arg
   1 5 10
<210> 7
   <211> 9
   <212> PRT
   <213> Homo sapiens
<400> 7
   Lys Tyr Lys Asp Tyr Phe Pro Val Ile
   1 5
<210> 8
   <211> 10
   <212> PRT
   <213> Homo sapiens
<400> 8
   Ala Ser Ser Ala Ser Ser Thr Leu Tyr Leu
   1 5 10
<210> 9
   <211> 9
   <212> PRT
   <213> Homo sapiens
<400> 9
   Ser Ser Ala Ser Ser Thr Leu Tyr Leu
   1 5
<210> 10
   <211> 9
   <212> PRT
   <213> Homo sapiens
<400> 10
   Leu Arg Thr Lys Val Tyr Ala Glu Leu
   1 5
<210> 11
   <211> 9
   <212> PRT
   <213> Homo sapiens
<400> 11
   Tyr Pro Pro Pro Pro Pro Ala Leu Leu
   1 5
<210> 12
   <211> 9
   <212> PRT
   <213> Homo sapiens
<400> 12
   Tyr Met Asp Gly Thr Met Ser Gln Val
   1               5
<210> 13
   <211> 9
   <212> PRT
   <213> Homo sapiens
<400> 13
   Val Pro Ser Gly Val Ile Pro Asn Leu
   1               5

## Beschreibung

Die vorliegende Erfindung betrifft bestimmte Melanom-assoziierte Oligopeptide, die von CD8-positiven cytotoxischen T-Lymphozyten (CTL) als Peptidantigen erkannt werden und die eine CTL-induzierte Lyse und/oder Apoptose von Tumorzellen herbeiführen. Weiterhin betrifft die vorliegende Erfindung die Verwendung dieser Melanom-assoziierten Oligopeptide in der Krebstherapie.

### Beschreibung

CD8-positive CTL stellen Effektorzellen des zellulären Immunsystems dar. Ihre Funktion besteht in der spezifischen Eliminierung von infizierten oder entarteten körpereigenen Zellen. Die CTL erkennen unter anderem tumorspezifische oder tumorassoziierte Peptidantigene (TAA), die an Haupthistokompatibilitätskomplex (MHC)-Moleküle der Klasse I gebunden und auf der Oberfläche der entarteten Zellen präsentiert sind. Die Erkennung der Peptidantigene im Kontext von MHC Klasse I-Molekülen erfolgt durch spezifische membranständige T-Zellrezeptoren (TCR) der CTL. Nach Erkennung wird die betreffende Zelle abgetötet, indem die CTL die Zielzellen lysieren und/oder den programmierten Zelltod (Apoptose) dieser Zielzellen induzieren oder Cytokine freisetzen. Die vorliegende Erfindung betrifft solche Tumor-assoziierte Peptide, die die Fähigkeit aufweisen, an ein Molekül des menschlichen Haupt-Histokompatibilitäts-Komplexes (MHC), Klasse I, zu binden. Derartige Peptide werden beispielsweise in der Immuntherapie von Tumorerkrankungen eingesetzt.

Bei der Eliminierung von Tumorzellen durch das Immunsystem spielt die Erkennung von Tumor-assoziierten Antigenen (TAA) durch Komponenten des Immunsystems eine herausragende Rolle. Diesem Mechanismus liegt die Voraussetzung zugrunde, dass zwischen Tumorzellen und normalen Zellen qualitative oder quantitative Unterschiede bestehen. Um eine anti-Tumor-Antwort herbeizuführen, müssen die Tumorzellen Antigene exprimieren, gegen welche eine immunologische Antwort erfolgt, die für die Eliminierung des Tumors ausreicht.

Zur Auslösung einer derartigen Immunreaktion durch cytotoxische T-Zellen müssen den T-Zellen dazu fremde Proteine/Peptide präsentiert werden. T-Zellen erkennen Antigene als Peptidfragmente nur dann, wenn diese an Zelloberflächen von MHC-Molekülen präsentiert werden. Diese MHC-Moleküle ("major histocompatibility complex") sind Peptidrezeptoren, die normalerweise Peptide innerhalb der Zelle binden, um sie zu der Zelloberfläche zu transportieren. Dieser Komplex aus Peptid und MHC-Molekül kann durch die T-Zellen erkannt werden. Die MHC-Moleküle des Menschen werden auch als humane Leukozyten-Anti gene-(HLA) bezeichnet.

Es gibt zwei Klassen von MHC-Molekülen: MHC-Klasse-I-Moleküle, die auf den meisten Zellen mit Kern vorkommen, präsentieren Peptide, die durch proteolytischen Abbau endogener Proteine entstehen. MHC-Klasse-II-Moleküle kommen nur auf professionellen Antigen-präsentierenden Zellen (APC) vor und präsentieren Peptide exogener Proteine, die im Verlauf der Endocytose von APC aufgenommen und verarbeitet werden. Komplexe aus Peptid und MHC-Klasse-I werden von CD8-positiven cytotoxischen T-Lymphozyten erkannt, Komplexe aus Peptid und MHC-Klasse-II werden von CD4-Helfer-T-Zellen erkannt.

Damit ein Peptid eine zelluläre Immunantwort auslösen kann, muss es an ein MHC-Molekül binden. Dieser Vorgang ist vom Allel des MHC-Moleküls und von der Aminosäuresequenz des Peptids abhängig. MHC-Klasse-I-bindende Peptide sind in der Regel 8-12 Reste lang und enthalten in ihrer Sequenz zwei konservierte Reste ("Anker"), die mit der entsprechenden Bindungsfurche des MHC-Moleküls interagieren.

Damit das Immunsystem eine effektive CTL-Antwort gegen Tumor-abgeleitete Peptide starten kann, müssen diese Peptide nicht nur in der Lage sein, an die bestimmten MHC-Klasse-I-Moleküle zu binden, die von den Tumorzellen exprimiert werden, sondern sie müssen auch von T-Zellen mit spezifischen T-Zell-Rezeptoren (TCR, "T-cell receptor") erkannt werden. Das Hauptziel zur Entwicklung eines Tumorvakzins ist die Identifizierung und Charakterisierung von Tumor-assoziierten Antigenen, die durch CD8⁺ CTL erkannt werden.

Die Antigene, die von den Tumor-spezifischen cytotoxischen T-Lymphozyten erkannt werden, bzw. deren Epitope, können Moleküle aus sämtlichen Proteinklassen sein, wie beispielsweise Enzyme, Rezeptoren, Transkriptionsfaktoren, usw. Eine andere wichtige Klasse Tumor-assoziierter Antigene sind Gewebe-spezifische Strukturen, wie beispielsweise CT ("cancer testis")-Antigene, die in verschiedenen Tumorarten und in gesundem Hodengewebe exprimiert werden.

Zu den tumorassoziierten Peptidantigenen, die im Kontext von MHC Klasse I-Molekülen auf der Oberfläche von Tumorzellen präsentiert werden, gehören zum Beispiel diejenigen, wie beschrieben in WO 95/25739 A1 für MAGE-3; in US 6,660,276 für Melanome und Tyrosinase und in EP 0 668 350 B1 für Melanome und gp100.

TAAs stellen somit ein Ausgangspunkt für die Entwicklung eines Tumorvakzine dar. Die Methoden zur Identifizierung und Charakterisierung der TAAs beruhen zum einen auf dem Einsatz von in Patienten bereits induzierten CTL oder basieren auf der Erstellung differentieller Transkriptionsprofile zwischen Tumoren und Normalgeweben.

Bisherige Impfstudien beschränkten sich auf die Anwendung weniger Tumor-assoziierter Antigene aus den Kategorien der Krebsarten/Keimbahn und der melanosomalen Proteine. Dabei kamen vor allem Antigene zum Einsatz, die häufig in Tumorgewebe exprimiert werden und die durch in der Bevölkerung häufige HLA-Allele präsentiert wurden. Dabei wurde auch nicht überprüft, ob die in den Studien behandelten Patienten gegen die Impfantigene ein Immunantwort entwickeln können (siehe zum Beispiel Rosenberg, Yang and Restifo Nat Med 10:909, 2004).

Dieses Vorgehen berücksichtigt jedoch nicht die zu vermutende grundsätzliche Individualität der Interaktion zwischen Tumorgewebe und dem körpereigenen Immunsystem. Bei übertragbaren und vital gefährdenden Infektionen werden in der Gesamtpopulation im Verlauf von Selektionsprozessen dominante Antigene und präferentielle HLA-Restriktionsmoleküle für Immunantworten erkennbar, die wesentlich zur Überwindung der Infektion und anschließender dauerhafter Protektion beitragen. Solche konstant und wiederkehrend zu beobachtenden Reaktionsmuster sind bei der Interaktion mit Malignomen nicht zu erwarten. Gründe hierfür sind:
- der interindividuell variable Antigen-Phänotyp von Malignomen (selbst gleichen histologischen Ursprungs) bedingt durch genetische und epigenetische Ereignisse im Tumor,
- der ausgeprägte Polymorphismus von HLA-Allelen, und
- die Fähigkeit des individuellen T-Zellrepertoires, auf bestimmte Epitope zu reagieren.

Es sind mehrere Dutzend Tumorpeptidantigene bekannt, die von T-Zellen erkannt werden können (zum Beispiel unter http://www.cancerimmunity.org/peptidedatabase). Allerdings reichen sie bei weitem nicht aus, um die antitumorale T-Zellantwort bei Patienten zu erklären. In den von uns untersuchten Modellen waren maximal 11 % der antitumoralen T-Zellklone gegen bekannte Peptide gerichtet. Sie erkannten statt dessen individualspezifische mutierte Peptide oder bislang nicht bekannte Peptide aus strukturell normalen tumorassoziierten Antigenen, die über patienteneigene HLA-Allele präsentiert wurden. Dies belegt die hochgradig individuell ausgelegte Zusammensetzung des antitumoralen T-Zellrepertoires beim Menschen.

Vor diesem Hintergrund ist es Aufgabe der vorliegenden Erfindung, neue Aminosäuresequenzen von immunogenen Peptiden bereitzustellen, welche die Fähigkeit aufweisen, an Moleküle des menschlichen Haupt-Histokompatibilitäts-Komplexes (MHC)-Klasse-I zu binden.

In einem ersten Aspekt wird diese Aufgabe erfindungsgemäß durch ein Melanom-spezifisches Immunogen gelöst, das ein Peptid mit einer Länge von 10 bis 200 Resten ist und das die Aminosäuresequenz der SEQ ID Nr 2 umfasst wobei das Immunogen eine Melanom-spezifische HLA-restringierte CTL-Antwort hervorruft

Im Rahmen der Erfindung wurden bei zwei Melanompatienten (bezeichnet als D05-GS und D14-SJR) zwölf neue Epitope (in immunogenen Peptiden) aus bereits bekannten Tumorantigenen der Kategorien Cancer/Germline (=CG)- und melanosomale Proteine identifiziert, die von tumorreaktiven CD8⁺-T-Zellen aus Blut der Patienten auf deren eigenen Tumorzellen und in Assoziation mit den bei ihnen vorliegenden HLA-Klasse I-Allelen erkannt werden. Dabei wurden zwei mutierte Antigene gefunden, die von Genen mit tumorspezifischen "Missense"-Mutationen kodiert werden. Im Gefolge der Mutation entstehen immunogene Peptide, die von den T-Zellen des jeweiligen Patienten erkannt werden. In der untenstehenden Tabelle 1 sind die Peptide angegeben, die von T-Zellen in niedrigster Konzentration erkannt werden, sowie die HLA-Allele, von denen sie präsentiert werden.

Im Rahmen der Erfindung werden die Ausdrücke "Immunogen", "immunogene Oligopeptide", "Oligopeptide" und "Peptide" austauschbar verwendet.

Die identifizierten Immunogene umfassen die Peptidsequenzen WQYFFPVIF (SEQ ID Nr. 1; HLA-Cw*020202-restringiert); KRCFPVIFGK (SEQ ID Nr. 2; HLA-B*270502-restringiert); KVDELAHFL (SEQ ID Nr. 3; HLA-Cw*0501-restringiert); NMVPFFPPV (SEQ ID Nr. 4; HLA-A*020101-restringiert); MPREDAHFIY (SEQ ID Nr. 5; HLA-B*510101-restringiert); LYPEWTEAQR (SEQ ID Nr. 6; HLA-A*24020101-restringiert); KYKDYFPVI (SEQ ID Nr. 7; HLA-Cw*0702 oder HLA-A*24020101-restringiert); ASSASSTLYL (SEQ ID Nr. 8; HLA-Cw*150201-restringiert), SSASSTLYL (SEQ ID Nr. 9; HLA-Cw*150201-restringiert) und VPSGVIPNL (SEQ ID Nr. 13; HLA-B*070201-restringiert). Weiterhin wurden zwei gegenüber der WildtypSequenz mutierte TAA-Epitope identifiziert (mutierte Aminosäure unterstrichen), nämlich LRTKVYAEL (SEQ ID Nr. 10; HLA-B*270502-restringiert) und YPPPPPALL (SEQ ID Nr. 11; HLA-B*510101-restringiert).

Mit Hilfe der erfindungsgemäßen Immunogene/Oligopeptide können cytotoxische T-Zellen generiert werden, die eine Antigen-spezifische MHC-restringierte cytotoxische Aktivität gegen die die erfindungsgemäßen Immunogene/Oligopeptide exprimierenden Tumorzellen entwickeln und diese zerstören.

Somit eröffnen diese Peptide die Möglichkeit einer wirkungsvollen Tumortherapie, bei der die in Tumorpatienten häufig beobachtete Unterdrückung einer Immunreaktion gegen Tumorzellen umgekehrt werden kann.

Wie den Ausführungsbeispielen zu entnehmen ist, ist es den Erfindern gelungen, nachzuweisen, dass gegen die erfindungsgemäßen Oligopeptide sehr wirksam cytotoxische T-Zellen erzeugt werden können, die die Tumorzellen, die entsprechende TAA herstellen und auf ihrer Oberfläche tragen, in einer MHC-Klasse-I-restringierten Weise abtöten. Diese Tumorzellen präsentieren nämlich über MHC-Klasse-I-Moleküle bestimmte Fragmente sämtlicher Proteine, die von ihnen hergestellt werden. Erkennen nun cytotoxische T-Zellen das von einem MHC-Klasse-1-Molekül präsentierte Peptid, durch das sie ursprünglich aktiviert wurden, so töten sie diese Zelle ab. Somit bietet dieses Peptid, das von MHC-Klasse-I-Molekülen TAA exprimierter Zellen präsentiert wird, die vorteilhafte Möglichkeit, gezielt eine Immunreaktion gegen Tumore auszulösen, die entsprechende TAA herstellen.

Erfindungsgemäß bevorzugt ist, dass die erfindungsgemäßen Immunogene eine Länge von 9 bis 11 Resten aufweisen. Dabei können N- und/oder C-terminal von der "Core-Region" der SEQ ID Nr 2 weitere Aminosäuren vorhanden sein. Diese weiteren Aminosäuren sind bevorzugt ausgewählt aus der Ursprungssequenz des entsprechenden TAA, können jedoch auch aus den anderen bekannten 20 Aminosäuren ausgewählt sein. Entsprechend können bevorzugte Peptide 200, Aminosäuren lang sein, ohne jedoch mit der Gesamtsequenz der jeweiligen entsprechenden TAA (also z.B. MAGE, gp100, usw., siehe Tabelle 1) übereinzustimmen. Insbesondere sind diese verlängerten Peptide für die externe Beladung von Zellen geeignet, und werden (ohne an diese Theorie gebunden sein zu wollen) zu einer Präsentation weiter prozessiert. Weiter bevorzugt sind Peptide mit einer Länge von 9, 10, 11, 12, 13, 14, 15, 16, 17, 18, 19 oder 20 Aminosäuren. Solche verlängerten Immunogene werden erfindungsgemäß ebenfalls von dem Begriff "Oligopeptide" umfasst.

Die Offenbarung betrifft auch ein Fusionsprotein, das aus einem der vorstehend beschriebenen Oligopeptide und aus einem weiteren Peptid oder Protein zusammengesetzt ist und welches zur Verwendung bzw. für den Einsatz als Diagnostikum oder Therapeutikum oder Prophylaktikum oder allgemein für eine Detektion und/oder Manipulation von T-Zellen, die eines der in den SEQ ID Nrn.: 1 bis 12 dargestellten Oligopeptide erkennen, geeignet ist. Möglich sind z.B. Fusionsproteine, die aus einem Trägerprotein, wie z.B. HSA, Collagen oder anderen Proteinen und einem oder mehreren der Oligopeptide der Erfindung bestehen. Diese Fusionen können auch eines oder mehrere der erfindungsgemäßen Epitope als Aminosäure-Kassetten enthalten. Die für dieses Fusionsprotein kodierenden Polynukleotide sind ebenfalls Gegenstand der vorliegenden Erfindung.

Auch modifizierte Formen der Peptide der SEQ ID Nrn.: 1 bis 12 können zu der gewünschten Immunantwort führen.

Dabei wird unter "modifiziert" im Sinne der Offenbarung jede chemische, enzymatische oder sonstige Veränderung des Peptides verstanden. Dies kann bspw. schon bei der Herstellung des Peptides oder später durch das Entfernen oder Hinzufügen einzelner Aminosäure-Reste, den Austausch einzelner Aminosäure-Reste, aber auch durch die chemische Veränderung einzelner Aminosäure-Reste durch das Anhängen bestimmter chemischer Gruppen erfolgen.

Ein weiterer Aspekt der Erfindung betrifft ein erfindungsgemäßes Immunogen, wobei das Immunogen eine durch Aminosäure-Substitution, -Deletion oder -Insertion, einer einzigen Aminosäure davon ableitbare Aminosäuresequenz aufweist, die ein funktionelles Äquivalent zu der Aminosäuresequenz zu einem Peptid der SEQ ID Nr. 2 darstellt, und es ein Epitop für CD8-positive CTL darstellt und dazu geeignet ist, eine auf humanes Leukozyten-Antigen der Molekülgruppe MHC Klasse I, Allelvariante A oder B restringierte Immunantwort von CD8-positiven CTL gegen Tumorzellen zu induzieren. Ein noch weiterer Aspekt der Erfindung betrifft ein Retro-inverses Peptid oder Pseudopeptid, **dadurch gekennzeichnet, dass** es einem Immunogen einem der Ansprüche 1 bis 4 entspricht, bei dem anstelle der -CO-NH-Peptidbindungen -NH-CO-Bindungen ausgebildet sind (vgl. z.B. Meziere C, Viguier M, Dumortier H, Lo-Man R, Leclerc C, Guillet JG, Briand JP, Muller S. In vivo T helper cell response to retro-inverso peptidomimetics. J Immunol. 1997 Oct 1;159(7):3230-7.).

Besonders bevorzugt ist ein erfindungsgemäßes Immunogen, wobei das Immunogen mit dem Peptid der SEQ ID Nr. 2 identisch ist.

Die Offenbarung betrifft auch ein Fusionsprotein, welches bevorzugt aus einem der vorstehend beschriebenen Oligopeptide, aus einer schweren Kette des HLA-Moleküls und aus einem flexiblem Linker besteht und derart konstruiert ist, dass das Oligopeptid derart aufgebaut (geeignet oder in der Lage bzw. befähigt) ist, die Peptid-Bindungsfurche des HLA-Moleküls zu besetzen, und welches zur Verwendung bzw. für den Einsatz als Diagnostikum oder Therapeutikum oder Prophylaktikum oder allgemein für eine Detektion und/oder Manipulation von T-Zellen, die eines der in den SEQ ID Nrn.: 1 bis 12 dargestellten Oligopeptide erkennen, geeignet ist. Die für dieses Fusionsprotein kodierenden Polynukleotide sind ebenfalls Gegenstand der vorliegenden Offenbarung.

In einer weiteren bevorzugten Ausgestaltung betrifft die Erfindung ein Polynukleotid, umfassend eine Nukleotidsequenz, die einen Sequenzabschnitt umfasst, der für mindestens ein erfindungsgemäßes Immunogen kodiert. Darüber hinaus weist die Nukleinsäure gegebenenfalls weitere Sequenzen auf, die zur Expression der dem Peptid entsprechenden NukleinsäureSequenz notwendig sind. Die verwendete Nukleinsäure kann in einem Vektor enthalten sein, der geeignet ist, die Expression der dem Peptid entsprechenden Nukleinsäuresequenz in einer Zelle zu ermöglichen.

Eine derartige Nukleinsäure hat den Vorteil, dass sie chemisch stabiler und unempfindlicher ist als Peptide. Die Handhabung ist daher einfacher als die der Peptide, und die Lagerfähigkeit von Nukleinsäuren ist nahezu unendlich. Sie sind chemisch und/oder molekularbiologisch sehr günstig und im Prinzip in unbegrenzten Mengen herzustellen. Eventuell zur Expression notwendige Nukleinsäuresequenzen haben ebenso wie ein Vektor, der die Nukleinsäuren enthält, den Vorteil, dass es dadurch möglich ist, große Mengen der Peptide sehr kostengünstig durch zelluläre Expressionssysteme mit Hilfe der Nukleinsäuren herzustellen.

Die Nukleinsäuren können aber auch dazu verwendet werden, Antigen-präsentierende Zellen, insbesondere dendritische Zellen, so zu transformieren, dass sie die entsprechenden Peptide selbst herstellen und dann mittels MHC-I-Molekülen cytotoxischen T-Zellen bzw. deren Vorläuferzellen präsentieren. Vorteilhaft ist dabei, dass die Präsentation der entsprechenden Peptide durch die Antigen-präsentierenden Zellen längerfristig erfolgt als bei der Präsentation von Peptiden, die lediglich von außen angeboten werden.

Ein weiterer Aspekt der vorliegenden Erfindung ist eine pharmazeutische Zusammensetzung zur *in vivo* oder *in vitro* Aktivierung von T-Zellen, insbesondere CD8-positiven cytotoxischen T-Lymphozyten, die mindestens ein erfindungsgemäßes Immunogen und/oder mindestens ein erfindungsgemäßes retro-inverses Peptid oder Pseudopeptid und/oder mindestens ein erfindungsgemäßes Polynukleotid umfaßt, gegebenenfalls mit entsprechenden akzeptablen Trägern und Hilfsstoffen.

Ein noch weiterer Aspekt der vorliegenden Erfindung ist ein rekombinantes DNA- oder RNA-Vektormolekül, das mindestens eines oder mehrere erfindungsgemäße Polynukleotid(e) enthält und das in Zellen autologen, allogenen, xenogenen oder mikrobiologischen Ursprungs exprimierbar ist. Ein weiterer Aspekt der vorliegenden Erfindung ist dann eine entsprechende Wirtszelle, die ein solches Polynukleotid oder ein solches Vektormolekül enthält.

Ein weiterer Aspekt der Offenbarung betrifft eine pharmazeutische Zusammensetzung, die ein erfindungsgemäßes Peptid oder eine erfindungsgemäße Nukleinsäure in einer eine MHC-1-restringierte Immunantwort auslösenden wirksamen Menge enthält.

Dazu können die Peptide und/oder Nukleinsäuren in den jeweils angemessenen üblichen Galeniken zubereitet sein. Im Falle von Peptiden könnten dies z.B. Zubereitungen sein, die üblicherweise für Impfungen verwendet werden, und ein Adjuvans enthalten. Im Falle von Nukleinsäuren ist auch eine Zubereitung mit Liposomen oder Vesikeln möglich. Durch eine entsprechende pharmazeutische Zubereitung ist es möglich, Organismen direkt zu behandeln, ohne ihnen vorher Antigen-präsentierende Zellen bzw. deren Vorläuferzellen entnehmen zu müssen, um diese zunächst zu züchten und sie nach Behandlung mit Peptiden oder Nukleinsäuren in den Patienten einzubringen. Mittels einer entsprechenden pharmazeutischen Zubereitung kann eine Tumorbehandlung in Form einer Impfung erfolgen.

Ein weiterer Aspekt der Offenbarung betrifft die Verwendung einer erfindungsgemäßen Nukleinsäure im Rahmen einer Gentherapie.

Dabei kann die Gentherapie in Form der oben bereits beschriebenen Transformation von Antigen-präsentierenden Zellen, insbesondere dendritischen Zellen, erfolgen, die oder deren Vorläuferzellen zuvor aus dem Körper eines zu behandelnden Organismus entnommen wurden, um nach der Transformation wieder in den Körper eingebracht zu werden. Gegenüber der Verwendung von Peptiden ist dabei, wie bereits erläutert, die länger anhaltende Präsentation von Vorteil.

Eine weitere Möglichkeit besteht darin, die Nukleinsäure so in den Körper einzubringen, dass sie selektiv von Antigen-präsentierenden Zellen, insbesondere dendritischen Zellen, aufgenommen und exprimiert wird. Diese Verwendung hat den Vorteil, dass außer der Verabreichung keine weiteren Maßnahmen, wie zum Beispiel die Anzucht und selektive Vermehrung entnommener dendritischer Zellen bzw. deren Vorläuferzellen, notwendig sind.

Die Offenbarung betrifft ferner die Verwendung einer erfindungsgemäßen Nukleinsäure zur Transformation oder Transfektion von Zellen *in vitro.* Der Einsatz der Nukleinsäure in vitro hat den Vorteil, dass Verfahren, wie zum Beispiel die Elektroporation und/oder Hilfstoffe wie bspw. Calciumphosphat oder DEAE-Dextran, verwendet werden können, die die Aufnahme von Nukleinsäuren in Zellen wesentlich erleichtern und verbessern, die aber in vivo nicht einsetzbar sind.

Dabei können, wie bereits erwähnt, Antigen-präsentierende Zellen, insbesondere dendritische Zellen, behandelt werden, die zum Beispiel deren Vorläuferzellen aus einem Patienten entnommen wurden und die später wieder in den Patienten eingebracht werden.

Ein weiterer Aspekt der vorliegenden Offenbarung ist die Verwendung mindestens eines erfindungsgemäßen Peptides oder einer erfindungsgemäßen Nukleinsäure zur Auslösung einer Immunreaktion im Zusammenhang mit einer Tumortherapie oder mit einer gegenüber der Entstehung eines Tumors vorbeugenden Behandlung. Vorteilhaft ist dabei, dass die bei einer Tumorerkrankung häufig beobachtete Immunsuppression und Toleranz gegenüber TAA durch die Verwendung der Peptide oder Nukleinsäuren umgekehrt werden kann. Darüber hinaus kann die Verwendung zusätzlich zu etablierten Tumortherapien eingesetzt werden.

Eine vorbeugende Behandlung ist möglicherweise vor allem bei Personen vorteilhaft, die, bspw. erblich bedingt oder weil sie früher bereits einen Tumor hatten, ein erhöhtes Risiko haben, an einem Tumor zu erkranken.

In einer Weiterbildung wird mindestens eines der erfindungsgemäßen Peptide oder Nukleinsäuren zusammen mit Antigen-präsentierenden Zellen, insbesondere dendritischen Zellen, inkubiert und erst dann in einen Organismus eingebracht, aus dem die Antigen-präsentierenden Zellen oder deren Vorläuferzellen zuvor entnommen wurden.

Der Vorteil dieses Verfahrens besteht darin, dass der Erfolg beim Auslösen einer Immunreaktion auf diese Art und Weise gesicherter und kontrollierbarer ist als bei der Injektion eines Peptides zusammen mit einem Adjuvans, bei der die Immunreaktion einmal stärker und einmal schwächer ausfallen kann. Gerade bei einer Tumortherapie sollte man jedoch den Erfolg beim Auslösen einer Immunreaktion sicherstellen können, um nicht durch eine uneffektive Behandlung möglicherweise wertvolle Zeit zu verlieren.

Die Oligopeptide (SEQ ID Nrn.: 1 bis 12 und deren Derivate) können in der aktiven und passiven Immunisierung von Patienten mit Melanomen, bei denen die entsprechenden Epitope der SEQ ID Nrn.: 1 bis 12 präsentiert wird/werden, eingesetzt werden, um die Induktion, Generierung und Expandierung von spezifischen cytotoxischen 1-Lymphozyten herbeizuführen, die in der Lage sind, die Tumorzellen der betreffenden Patienten spezifisch abzutöten und dadurch eventuelle eine Heilung zu vermitteln und/oder zu unterstützen.

Die Derivate der Oligopeptide der SEQ ID Nrn.: 1 bis 12 und auch die davon abgeleiteten retro-inversen Peptide oder Pseudopeptide haben gegenüber dem jeweils ursprünglichen Oligopeptid selbst den Vorteil, dass damit eine potentielle funktionelle Selbsttoleranz (gegenüber der Oligopeptide der SEQ ID Nrn.: 1 bis 12) auf T-Zellebene umgangen werden kann. Während das Oligopeptid aufgrund der (geringen) Expression in einigen Normalgeweben unter Umständen in dem betreffenden Organismus (Patientenkörper) ein so genanntes Tolerogen darstellt und für die organismuseigenen (patienteneigenen) CTL nicht immunogen ist, werden die Derivate dieser Oligopeptide (SEQ ID Nrn.: 1 bis 12) im Regelfall als Antigene erkannt und induzieren die Aktivierung und Expandierung von CTL. Diese Derivat-induzierten CTL weisen in der Regel eine hohe Kreuzreaktivität gegenüber der betreffenden SEQ ID Nrn.: 1 bis 12 Wildtypsequenz auf und induzieren infolgedessen auch die Lyse und/oder Apoptose von solchen (Tumor-)Zellen, die SEQ ID Nrn.: 1 bis 12 (im Kontext von HLA auf ihrer Oberfläche präsentieren.

Besonders bevorzugte Derivate der Oligopeptide der SEQ ID Nrn.: 1 bis 12 sind solche, die natürlicherweise in anderen Säuge- oder Wirbeltieren vorkommen, zum Beispiel SEQ ID Nrn.: 1 bis 12-Homologe aus der Maus. Die SEQ ID Nrn.: 1 bis 12 (Protein-) und Peptid-Homologe und die dafür kodierenden Nukleinsauren können relativ leicht, nämlich direkt und mit geläufigen Isolationsverfahren aus dem jeweiligen Organismus gewonnen werden.

Die Oligopeptide SEQ ID Nrn.: 1 bis 12 und deren Derivate sowie die retro-inversen Peptide oder Pseudopeptide können mittels gängiger Peptidsyntheseverfahren hergestellt werden, und die für diese Oligopeptide kodierenden Nukleotidsequenzen können mit bekannten chemischen oder mit molekularbiologischen Verfahren gewonnen werden.

Es besteht zudem die Möglichkeit, aus den vorstehend beschriebenen Oligopeptiden, einem flexiblem Linker und einer schweren Kette des HLA-Moleküls ein Fusionsprotein zu konstruieren, und zwar derart, dass das Oligopeptid dazu befähigt (in der Lage bzw. geeignet) ist, die Peptid-Bindungsfurche des HLA-Moleküls zu besetzen. Diese Fusionsproteine und dafür kodierende Polynukleotide eignen sich insbesondere als (Wirkstoff von einem) Diagnostikum oder Therapeutikum oder Prophylaktikum oder allgemein für eine Detektion und/oder Manipulation von T-Zellen, die eines der in den SEQ ID Nrn.: 1 bis 12 dargestellten Oligopeptide erkennen.

Die Oligopeptide (SEQ ID Nrn.: 1 bis 12 und deren Derivate) sowie die retro-inversen Peptide oder Pseudopetide und die vorstehend beschriebenen Fusionsproteine eignen sich sowohl zur in-vivo-Induktion von T-Lymphozyten im Patienten als auch zur in-vitro-Induktion und -Expansion entsprechend reaktiver patienteneigener oder patientenfremder T-Lymphozyten.

Für eine in vivo Induktion und -Expansion von T-Lymphozyten im Patienten kommen verschiedene Verfahren in Betracht, beispielsweise (a) die Injektion der erfindungsgemäßen Oligopeptide und/oder eines oder mehrerer Derivate eines oder beider dieser Oligopeptide und/oder eines retro-inversen Peptids oder Pseudopeptids und/oder eines vorstehend beschriebenen Fusionsproteins als reines Peptid oder zusammen mit Adjuvantien oder mit Cytokinen oder in einem geeigneten Freisetzungssystem wie z.B. Liposomen, (b) die Injektion einer oder mehrerer mindestens der erfindungsgemäßen Oligopeptide oder deren Derivate und/oder für eines der retro-inversen Peptide oder Pseudopeptide und/oder für eines der Fusionsproteine kodierenden Nukleinsäuren in nackter oder komplexierter Form oder in Form von viralen oder nicht-viralen Vektoren oder zusammen mit Freisetzungssystemen wie kationischen Lipiden oder kationischen Polymeren, (c) die Beladung von Zellen autologen, allogenen, xenogenen oder mikrobiologischen Ursprungs mit den erfindungsgemäßen Oligopeptiden oder deren Derivaten oder dazu analogen retro-inversen Peptiden oder Pseudopeptiden, (d) die Beladung von Zellen autologen, allogenen, xenogenen oder mikrobiologischen Ursprungs mit dem erfindungsgemäßen Oligopeptiden oder Homologen anderer Spezies, so dass infolgedessen das erfindungsgemäße Oligopeptid und/oder Derivate eines oder beider dieser Oligopeptide auf den jeweiligen Zellen präsentiert wird, oder (e) die Transfektion oder Infektion von Zellen autologen, allogenen, xenogenen oder mikrobiologischen Ursprungs mit den mindestens für das erfindungsgemäße Oligopeptid oder deren Derivate oder für ein davon abgeleitetes retro-inverses Peptid oder Pseudopeptid oder für ein vorstehen beschriebenes Fusionsprotein kodierenden Nukleinsäuren (wiederum entweder in "nackter" oder komplexierter Form oder in Form von viralen oder nicht-viralen Vektoren).

Im Fall einer in-vitro-Induktion und -Expansion werden die in-vitro gewonnenen T-Lymphozyten anschließend dem Patienten per Infusion oder Injektion oder ähnlichen Verfahren zugeführt.

Die Erfindung betrifft deshalb auch die Verwendung des erfindungsgemäßen Oligopeptide und/oder deren Derivate und/oder dazu analoger retro-inverser Peptide oder Pseudopeptide zur Herstellung von Diagnostika und/oder Prophylaktika und/oder Therapeutika (insbesondere Impfstoffe) für den Nachweis und/oder die Beeinflussung und/oder Generierung und/oder Expandierung und/oder Steuerung des Aktivierungs- und Funktionszustands von T-Zellen, insbesondere CD8-positiven CTL.

Als Therapeutika und/oder Prophylaktika kommen insbesondere Impfstoffe oder Injektionen oder Infusionslösungen in Betracht, die als Wirkstoff (a) das erfindungsgemäße Oligopeptid und/oder mindestens ein Derivat eines dieser Oligopeptide und/oder mindestens ein zu einem dieser Oligopeptide oder zu deren Derivate analoges retro-inverses Peptid oder Pseudopeptid und/oder mindestens eines der vorstehend beschriebenen Fusionsproteine enthalten, und/oder die (b) eine Nukleinsäure enthalten, die mindestens für das erfindungsgemäße Oligopeptid oder mindestens für ein Derivat eines dieser Oligopeptide kodiert, und/oder die (c) in-vitro erzeugte T-Lymphozyten, welche spezifisch gegen das erfindungsgemäße Oligopeptid und/oder deren Derivat(e) und/oder gegen ein zu einem dieser Oligopeptide oder zu einem Derivat dieser Oligopeptide analoges retro-inverses Peptid oder Pseudopeptid gerichtet sind, enthalten.

Zur Herstellung der Diagnostika oder auch der Therapeutika oder auch der Prophylaktika eignen sich insbesondere auch rekombinante DNA- oder RNA-Vektormoleküle, die ein oder mehrere Polynukleotid(e) enthalten, welche für ein Oligopeptid der SEQ ID Nr.: 2 und/oder für mindestens ein Derivat dieses Oligopeptids kodieren, und die in Zellen autologen, allogenen, xenogenen oder mikrobiologischen Ursprungs transkribierbar bzw. exprimierbar sind. Die Erfindung umfasst deshalb auch solche rekombinanten DNA- oder RNA-Vektormoleküle und Wirtszellen, die diese Vektormoleküle enthalten.

Als Diagnostikum oder Therapeutikum oder Prophylaktikum oder allgemein für eine Detektion und/oder Manipulation von entsprechenden TAA überexprimierenden Zellen können erfindungsgemäß auch polyklonale, monoklonale oder rekombinante Antikörper eingesetzt werden, die gegen das Oligopeptid der SEQ ID Nr.: 2 und/oder gegen ein Derivat dieses Oligopeptids und/oder gegen ein zu einem dieses Oligopeptids oder dessen Derivate analoges retro-inverses Peptid oder Pseudopeptid gerichtet sind oder die mit einem Komplex aus einem der betreffenden Oligopeptide bzw. dessen Derivate bzw. dazu retro-inversen Peptid(en) und/oder Pseudopeptid(en) und HLA reagieren.

Die Verwendung des Oligopeptids der SEQ ID Nr.: 2 und/oder eines Derivates dieses Oligopeptids und/oder eines zu diesem Oligopeptid oder zu einem Derivat dieses Oligopeptids analogen retro-inversen Peptids oder Pseudopeptids für die Herstellung polyklonaler, monoklonaler oder rekombinanter Antikörper gegen ein solches erfindungsgemäßes Oligopeptid bzw. retro-inverses Peptid oder Pseudopeptid und der/die betreffende(n) Antikörper an sich sind folglich ebenfalls Teil der vorliegenden Erfindung.

Als Diagnostikum oder Therapeutikum oder Prophylaktikum oder allgemein für eine Detektion und/oder Manipulation von entsprechenden TAA überexprimierenden Zellen können erfindungsgemäß auch polyklonale, monoklonale oder rekombinante HLA-restringierte T-Zellrezeptoren oder dazu funktionell äquivalente Moleküle eingesetzt werden, die spezifisch für ein Oligopeptid der SEQ ID Nr.: 2 und/oder ein Derivat dieses Oligopeptids und/oder für dazu analoge retro-inverse Peptide oder Pseudopeptide sind. Die T-Zellrezeptoren oder dazu funktionell äquivalenten Moleküle können autologen, allogenen oder xenogenen Ursprungs sein.

Zum Gegenstand vorliegender Erfindung gehört folglich auch: die Verwendung eines Oligopeptids der SEQ ID Nr.: 2 und/oder eines Derivates dieses Oligopeptids und/oder dazu analoger retro-inverser Peptide oder Pseudopeptide oder die Verwendung von Polynukleotiden mit einer Nukleotidsequenz, die für ein Oligopeptid der SEQ ID Nr 2 und/oder ein Derivat dieses Oligopeptids kodiert, zur Herstellung polyklonaler, monoklonaler oder rekombinanter HLA-restringierter T-Zellrezeptoren oder dazu funktionell äquivalenter Moleküle mit Spezifität für ein solches erfindungsgemäßes Oligopeptid bzw. retro-inverses Peptid oder Pseudopeptid, der/die betreffende(n) T-Zellrezeptor(en) an sich und dazu funktionell äquivalente Moleküle, sowie Polynukleotide, die für diese T-Zellrezeptoren oder dazu funktionell äquivalenten Moleküle kodieren, Expressionsvektoren mit der Fähigkeit zur Expression dieser T-Zellrezeptoren oder dazu funktionell äquivalenten Moleküle, sowie entsprechende Wirtszellen, wie oben.

Die Offenbarung umfasst außerdem Reagenzien zur in-vivo- oder in-vitro-Aktivierung von T-Zellen, insbesondere CD8-positiven CTL, die dadurch gekennzeichnet sind, dass sie unter Verwendung eines Oligopeptids der SEQ ID Nrn.: 1 bis 12 und/oder wenigstens eines Derivates eines dieser Oligopeptide und/oder wenigstens eines dazu analogen retro-inversen Peptids oder Pseudopeptids oder wenigstens eines vorstehend beschriebenen Fusionsproteins und/oder unter Verwendung wenigstens eines Polynukleotids, das mindestens für das Oligopeptid oder dessen Derivat(e) kodiert und/oder unter Verwendung des entsprechenden TAA-Proteins oder dazu Homologen anderer Spezies, hergestellt sind. Bei diesen Reagenzien kann es sich insbesondere um Therapeutika, und dabei vor allem um Impfstoffe handeln.

Ein weiterer Aspekt der vorliegenden Erfindung ist ein Arzneimittel zur Behandlung von Erkrankungen, die mit einem der erfindungsgemäßen Immunogene der SEQ ID Nr.: 2 assoziiert sind, dadurch gekennzeichnet, dass das Arzneimittel mindestens ein Immunogen und/oder mindestens ein retro-inverses Peptid oder Pseudopeptid und/oder mindestens ein Polynukleotid und/oder mindestens einen T-Zellrezeptor und/oder mindestens ein Vektormolekül und/oder mindestens eine Wirtszelle und/oder mindestens ein Antikörper gemäß der Erfindung, gegebenenfalls zusammen mit geeigneten Zusatz- und Hilfsstoffen, enthält.

Ein weiterer Aspekt der vorliegenden Erfindung betrifft dann die Verwendung eines erfindungsgemäßen Immunogens und/oder eines erfindungsgemäßen retro-inversen Peptids oder Pseudopeptids zur Herstellung von Diagnostika und/oder Therapeutika und/oder Prophylaktika für den Nachweis und/oder die Beeinflussung und/oder Generierung und/oder Expandierung und/oder Steuerung des Aktivierungs- und Funktionszustands von T-Zellen, insbesondere CD8-positiven cytotoxischen T-Lymphozyten.

Ein noch weiterer Aspekt der vorliegenden Offenbarung betrifft dann die Verwendung mindestens eines erfindungsgemäßen Immunogens und/oder eines erfindungsgemäßen retro-inversen Peptids oder Pseudopeptids und/oder eines Fusionsproteins zur Auslösung einer Immunreaktion im Zusammenhang mit einer Tumortherapie oder mit einer gegenüber der Entstehung eines Tumors vorbeugenden Behandlung.

Zuletzt betrifft die vorliegende Offenbarung ein Verfahren zur Behandlung eines Patienten gegen Melanome, das Verabreichen einer therapeutisch effektive Menge mindestens eines Immunogens und/oder mindestens eines retro-inversen Peptids oder Pseudopeptids und/oder mindestens eines Fusionsproteins und/oder mindestens eines Polynukleotids und/oder mindestens eines T-Zellrezeptors und/oder mindestens eines Vektormoleküls und/oder mindestens einer Wirtszelle und/oder mindestens eines Antikörpers der Erfindung in einer Menge umfasst, wodurch ein therapeutischer Effekt erreicht wird. Ein weiterer Aspekt ist ein Verfahren zur Hervorrufung einer Melanom-spezifischen CTL-Antwort, umfassend das Verabreichen einer Antwort-hervorrufenden Menge des Melanom-spezifischen erfindungsgemäßen Immunogens, gegebenenfalls mit Hilfsstoffen, wie oben erwähnt.

Die Bereitstellung zusätzlicher definierter erfindungsgemäßer Immunogene ermöglicht, bei einem höheren Anteil von Patienten therapeutische Impfstudien durchzuführen. Damit verbunden erhöhen sich auch die Chancen auf den Einschluss individuell relevanter Antigene.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung soll nun im folgenden anhand von Ausführungsbeispielen unter Bezug auf die beigefügten Abbildungen näher dargestellt und erläutert werden, ohne jedoch darauf beschränkt zu sein.

**Tabelle 1: T-zellerkannte erfindungsgemäße Antigene in den Melanompatienten D05-GS und D14-SJR**

| **Peptidsequenz** | **Gen** | **restringierendes *HLA-Allel*** | **Antigenbezeichnung** | **SEQ ID Nr.** | **Verfahren** | **Prävalenz (%)** | **Patient** |
|---|---|---|---|---|---|---|---|
| WQYFFPVIF | MAGE-A3,-A6^{LICR} | HLA-Cw*020202 | MAGE-A3,6/Cw2 | 1 | 1 | 9-20 | D05-GS |
| KRCFPVIFGK | MAGE-A4 | HLA-B*270502 | MAGE-A4B27 | 2 | 1 | 3-4 | D05-GS |
| KVDELAHFL | MAGE-A4 | HLA-Cw*0501 | MAGE-A4/Cw5 | 3 | 2 | 4-8 | D05-GS |
| NMVPFFPPV | HSTRP-2 | HLA-A*020101 | HSTRP2/A2 | 4 | 4 | 41-43 | D05-GS |
| MPREDAHFIY | Melan-A | HLA-B*510101 | Melan-A/B51 | 5 | 1 | 4-37 | D14-SJR |
| LYPEWTEAQR | gp100 | HLA-A*24020101 | gp100/A24 | 6 | 1 | 17-20 | D14-SJR |
| KYKDYFPVI | MAGE-C2 | HLA-A*24020101 | MAGE-C2/A24 | 7 | 1/3 | 8-10/22-26 | D14-SJR |
| | | HLA-Cw*0702 | MAGE-C2/Cw7 | | | | |
| ASSASSTLYL | MAGE-C2 | HLA-Cw*150201 | MAGE-C2/Cw15(1) | 8 | 1 | 3-8 | D14-SJR |
| SSASSTLYL | MAGE-C2 | HLA-Cw*150201 | MAGE-C2/Cw15(1) | 9 | 1 | 3-8 | D14-SJR |
| LRTKVYAEL** | CCT6Amut | HLA-B*270502 | CCT6Amut/B27 | 10 | 4 | *Unbekannt* | D05-GS |
| YPPPPPALL** | N-WASPmut | HLA-B*510101 | N-WASPmut/B51 | 11 | 4 | *Unbekannt* | D14-SJR |
| YMDGTMSQV* | Tyrosinase | HLA-Cw*0501 | Tyr/Cw5 | 13 | 1 | 14-25 | D05-GS |
| VPSGVIPNL | MAGE-C2 | HLA-B*070201 | MAGE-C2B7 | 12 | 2 | 12 | D14-SJR |

| | | | | | | | |
|---|---|---|---|---|---|---|---|
| *: Peptid wurde in Verbindung mit HLA-A2 bereits publiziert; **: mutierte Aminosäure unterstrichen; Prävalenz: zu erwartende Prävalenz bei Patienten mit malignem Melanomen, ermittelt aus der Häufigkeit der Expression des jew. Antigens in Melanomen (laut Literatur) und der Häufigkeit, mit der das präsentierende HLA-Allel in nordeuropäischen und nordamerikanischen kaukasischen Bevölkerungsgruppen vorkommt (laut http://www.allelefrequencies.net/); Verfahren: 1: identifiziert durch Panel-Testung mit MLTC-Respondem; 2: identifiziert durch Panel-Testung mit klonalen T-Zellen; 3: identifiziert durch cDNA-Bank-Screening mit MLTC-Respondem; 4: identifiziert durch cDNA-Bank-Screening mit klonalen T-Zellen. | | | | | | | |

### Beispiele

Nachfolgend wird das methodische Vorgehen beschrieben, das zur Identifizierung der erfindungsgemäßen Melanom-Peptidantigene führte. Dabei wird ergänzend auch auf die beigefügten Abbildungen verwiesen.

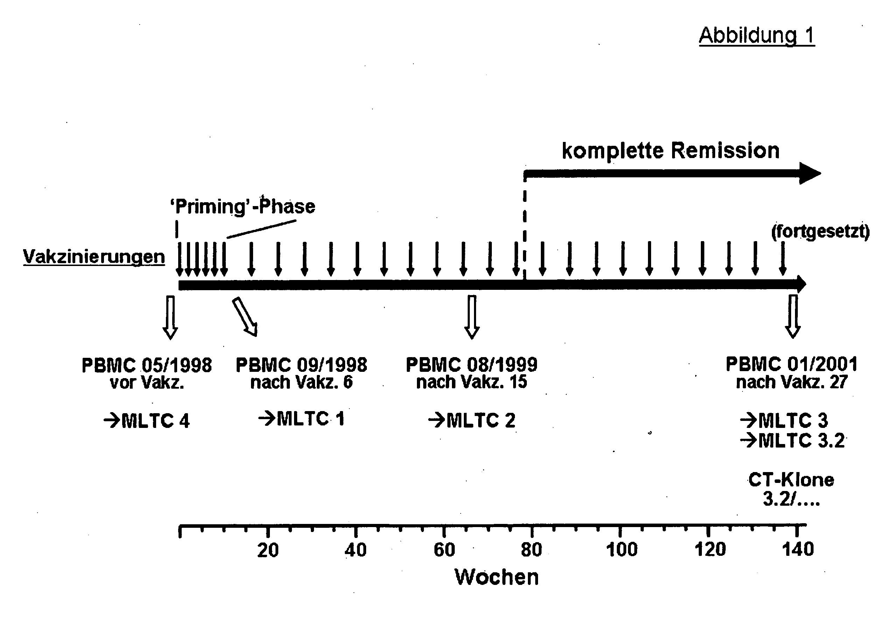

Aus den Tumoren der Patienten D05-GS und D14-SJR wurden stabil wachsende Melanomzelllinien etabliert. Zusätzlich wurden diesen Patienten, die an einer Impfstudie in Brisbane / Australien teilnahmen, über einen Zeitraum mehrerer Jahre Blut entnommen. **Abbildung 1** zeigt schematisch den Ablauf der Vakzinierungen, die Zeitpunkte der Blutentnahmen und die Herkunft der tumorreaktiven MLTC und T-Zellklone im Patientenmodell D05-GS. Analog geschahen die entsprechenden Schritte auch im Patientenmodell D14-SJR (nicht gezeigt).

Aus diesen Blutproben wurden in Brisbane Lymphozyten isoliert und kryokonserviert. Durch Prof. Pierre van der Bruggen vom Ludwig Institute for Cancer Research/Brüssel (abgekürzt LICR) wurde als Beitrag zur Erfindung ein Panel von cDNA-Klonen in Expressionplasmiden zur Verfügung gestellt, die für insgesamt 31 Antigene der Cancer/Germline (CG)-Kategorie und der Melanozyten-Differenzierungsantigen-Kategorie kodieren (**Tabelle 2**).

Die Erfinder klonierten aus der Melanomlinie des Patienten D05-GS mittels RT-PCR die HLA-Allele HLA-A*020101, HLA-B*270502, HLA-B*44020101, HLA-Cw*0501, HLA-Cw*020202, und aus der Melanomlinie des Patienten D14-SJR die HLA-Allele HLA-A*030101, HLA-A*24020101, HLA-B*070201, HLA-B*510101 und HLA-Cw*150201. Das vom Patienten D14-SJR ebenfalls getragene Allel HLA-Cw*0702 wurde den Erfindern im Rahmen einer Kooperation zur Verfügung gestellt. Aus den beiden Melanomlinien wurden cDNA-Banken in pcDNA3.1 konstruiert.

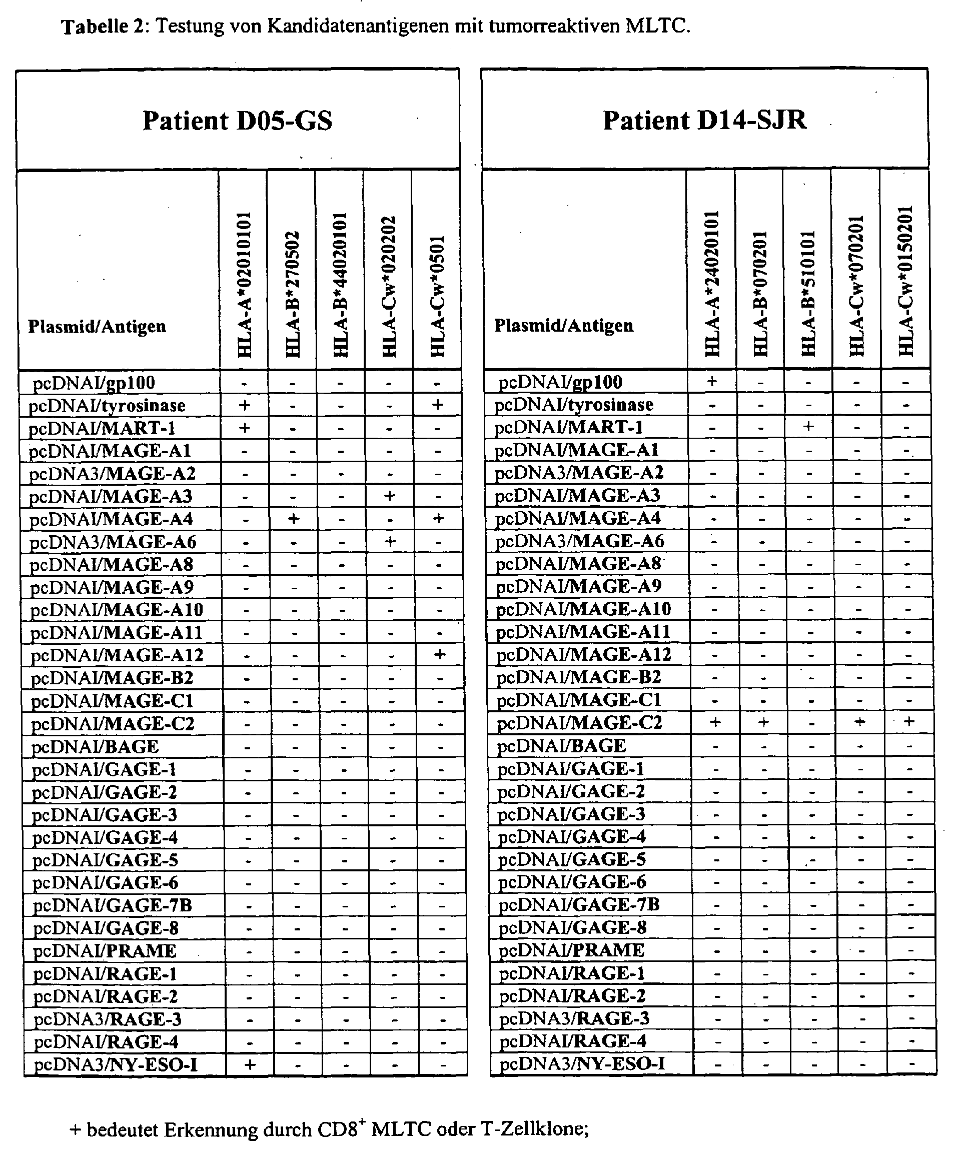

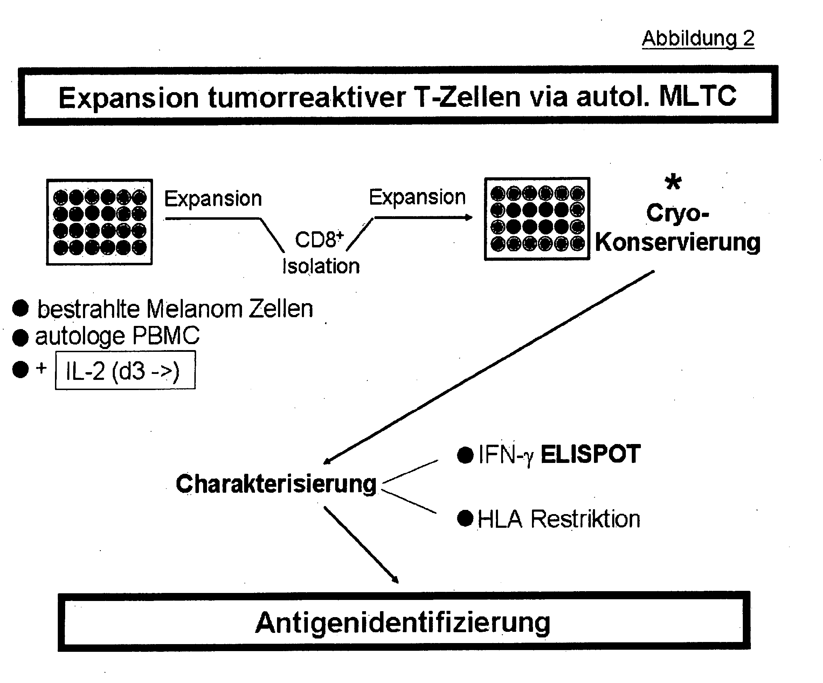

In so genannten gemischten Lymphozyten-/Tumorzellkulturen (MLTC) wurden Blut-Lymphozyten mit den jeweiligen vom gleichen Spender stammenden (autologen) Tumorzelllen stimuliert (**Abbildung 2**).

In den Responderlymphozyten wurden dadurch tumorspezifische T-Zellen angereichert. In jedem Patientenmodell wurden mehrere solcher MLTC-Responderpopulationen mit Blutlymphozyten verschiedener Jahre generiert und zu verschiedenen Zeitpunkten nach vorheriger Aufreinigung CD8-positiver T-Zellen kryokonserviert (**Abbildung 2**). Sie wurden vor Kryokonservierung auf die Erkennung autologer Melanom-Zellen und autologer EBVtransformierter B-Zellen hin überprüft. Darüber hinaus wurde durch Blockade der Tumorzellerkennung mit HLA-Gruppen-spezifischen Antikörpern die präferentielle HLA-Restriktion der MLTC-Responder ermittelt (nicht gezeigt).

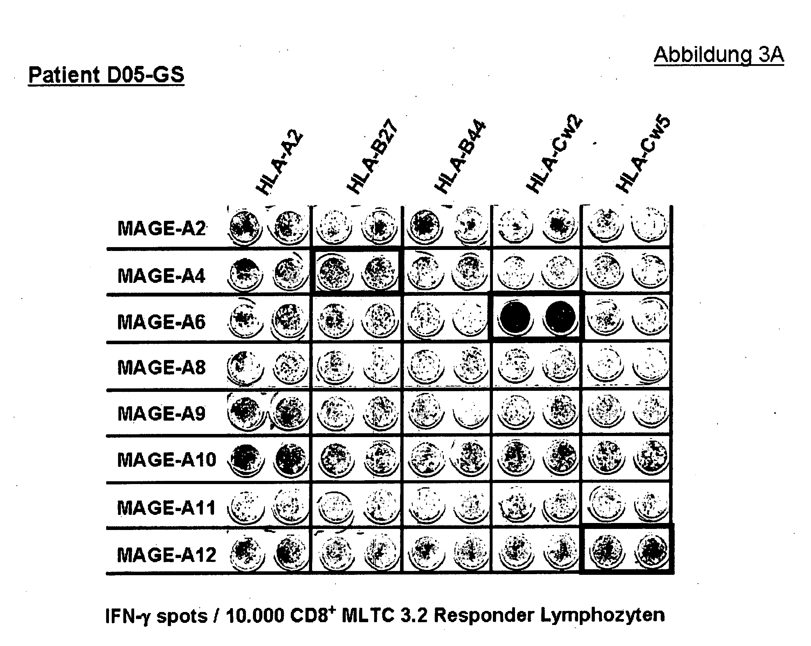

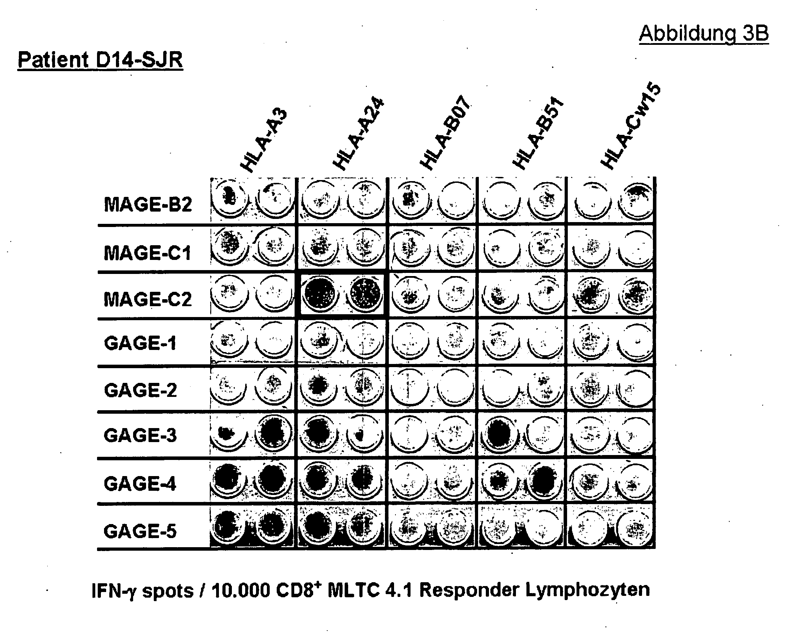

Zu verschiedenen Zeitpunkten kryokonservierte MLTC-Populationen wurden in einem ,Panel-Test' auf Reaktivität gegen bekannte Melanom-assoziierte Antigene überprüft. Dazu wurden die oben genannten Antigen-kodierenden Expressionsplasmide mit jedem der HLA-Allele der beiden Patienten in COS-7-Zellen oder 293T-Zellen transfiziert (**Tabelle 2**). Die Transfektanten wurden dann in Interferon-gamma-ELISPOT-Assays auf Erkennung durch MLTC-Responder getestet. Die **Abbildungen 3** zeigen je ein Beispiel aus den Testungen der MLTC 3.2 im Patientenmodell D05-GS (**Abb. 3A**) sowie der MLTC 4.1 aus dem Patientenmodell D14-SJR (**Abbildung 3B**). Insgesamt stellten die Erfinder eine T-Zellreaktivitäten gegen folgende, bisher nicht bekannte, Antigen-HLA-Kombinationen fest (siehe auch **Tabelle 2**):
**Patient (Modell) D05-GS:**
   Tyrosinase/HLA-Cw5; MAGE-A3/HLA-Cw2; MAGE-A6/HLA-Cw2; MAGE-A4/HLA-B27 und MAGE-A4/HLA-Cw5
**Patient (Modell) D14-SJR:**
   Melan-A/HLA-B51; gp100/HLA-A24; MAGE-A3/HLA-A24; MAGE-A6/HLA-A24; MAGE-C2/HLA-A24; MAGE-C2/HLA-B7; MAGE-C2/HLA-Cw7 und MAGE-C2/HLA-Cw15

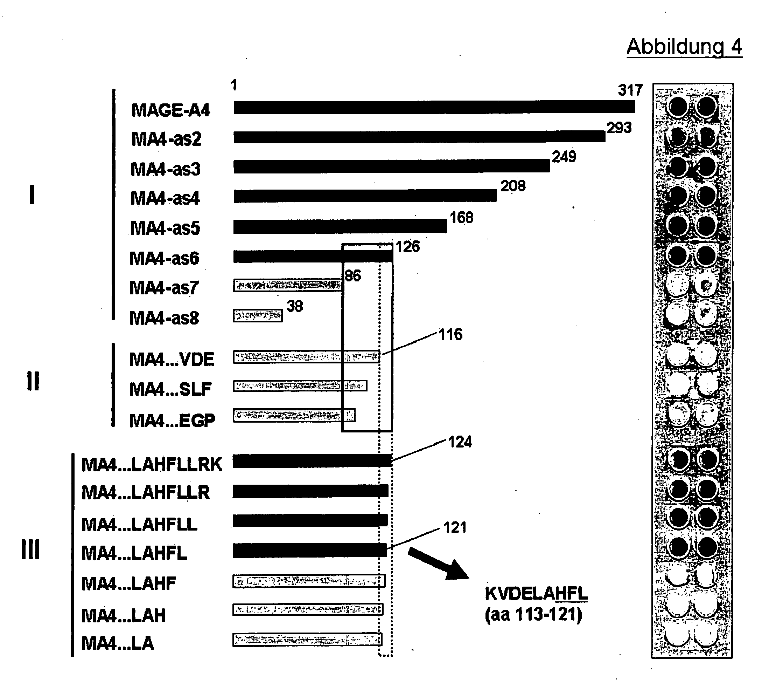

Im nächsten Schritt wurden die Peptid-kodierenden Genregionen für diese Antigene identifiziert. Dafür wurden Antigen-kodierende cDNAs mittels PCR fragmentiert, die Fragmente erneut in einen Expressionsvektor kloniert, mit den jeweiligen präsentierenden HLA-Allelen in COS-7- oder 293 T-Zellen kotransfiziert und im IFN-γ-ELISPOT-Test auf Erkennung durch die MLTC-Populationen oder T-Zellklone getestet. Erkannte Fragmente wurden weiter verkürzt und getestet bis der C-Terminus des erkannten Peptids identifiziert war. Die Abb. 4 zeigt z. B. die Identifizierung des MAGE-A4/HLA-Cw5-Peptids aus dem Patientenmodell D05-GS. Die Zahlen zeigen die Länge der von den cDNA-Fragmenten kodierten Polypeptidketten in Aminosäuren an. Das Peptid wurde in drei Schritten (I - III) identifiziert. Aus der Testung der ersten acht Fragmente konnte geschlossen werden, dass die Peptid-kodierende Region zwischen den Aminosäuren 86 und 126 lag (I). Die zweite Testung ergab, dass der C-Terminus des erkannten Peptids zwischen Aminosäuren 116 - 126 liegen musste (II). In der abschließenden Testung von Fragmenten, die für Polypeptide kodierten, die C-terminal sukzessive um eine Aminosäure verkürzt wurden, konnte der C-Terminus des erkannten Peptids auf die Aminosäure 121 festgelegt werden (III). Nach exakter Lokalisierung des C-terminalen Peptid-Endes wurden Peptide von 9 und 10 Aminosäuren Länge synthetisiert und auf Erkennung durch die T-Zellen überprüft. **Tabelle 1** gibt die auf diesem Weg identifizierten Peptide wieder.

Die antitumorale Reaktivität der MLTC-Responder überstieg die Summe der Reaktivitäten gegen bekannte Melanom-assoziierte Antigene. MLTC-Populationen wurden im Grenzverdünnungsverfahren kloniert und zytotoxische T-Zellklone ausgesucht, die zwar autologe Melanomzellen erkannten, aber keines der bekannten Antigene.

Solche Klone wurden für das cDNA-Bank-Screening zur Suche nach neuen Antigenen ausgewählt. Die cDNA-Bibliotheken wurden in Pools zu je 100 cDNA-Klonen aufgeteilt. Aus 2.000 solcher 100er-Pools wurden Plasmide extrahiert. Die cDNA-Pools wurden mit jedem der HLA-cDNAs des jeweiligen Modellsystems in COS-7- bzw. 293T-Zellen kotransfiziert und die Transfektanten im IFN-γ-ELISPOT-Assays auf Erkennung durch die T-Zellen getestet. Aus positiven 100er-Pools wurden antigen-kodierende cDNAs kloniert. Die Inserts der Klone wurden sequenziert. Zum Vergleich wurden Sequenzen aus Datenbanken herangezogen, sowie Sequenzen homologer cDNA aus autologen EBV-B-transformierten B-Zellen beider Patienten, die mittels RT-PCR generiert wurde.

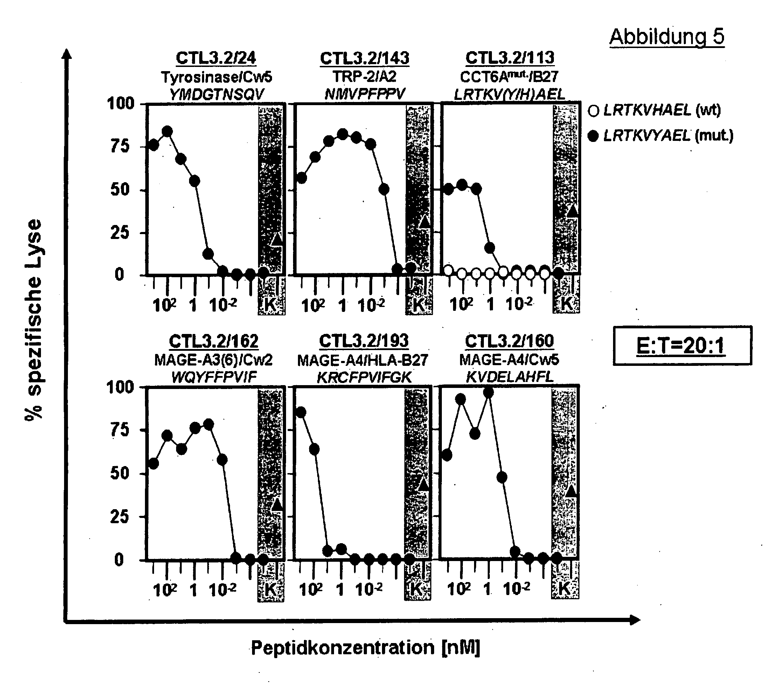

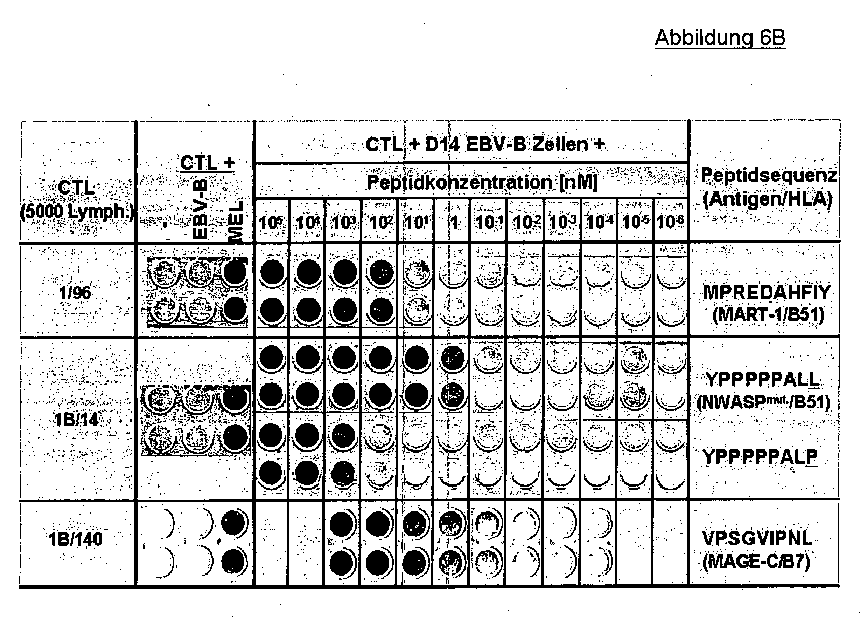

Bei zwei Antigenen (N-WASP im Modell D14-SJR und CCT6A (TCP20 ist ein Synonym für CCT6A) im Modell D05 wurden tumorspezifische Punktmutationen gefunden. Synthetische Peptide, die diese Mutationen enthielten und laut verfügbarer Peptidalgorithmen an die präsentierenden HLA-Allele banden, wurden synthetisiert und auf T-Zellerkennung überprüft. In beiden Fällen wurden die mutierten Peptide erkannt, nicht aber das homologe nicht-mutierte Peptid (CCT6A; **Abbildung 5**) bzw. das Wildtyp-Peptid 1000-fach schlechter als das mutierte (N-WASP, **Abbildung 6B**). Somit generierten die tumorspezifischen Neomutationen immunogene Peptide. Es ist bislang nicht bekannt, ob auch andere Melanome Mutationen von N-WASP und CCT6A aufweisen. Ein weiteres beim Bank-Screening gefundenes Antigen, TRP-2, entspricht einem strukturell normalen trielanosomalen Differenzierungsantigen, das zudem als T-Zellantigen bekannt ist. Die Erfinder fanden durch cDNA-Fragmentierung ein neues Peptid, das durch HLA-A2, dem häufigsten HLA-Klasse I-Allel der kaukasischen Bevölkerung, präsentiert wird (siehe **Tabelle 1**).

**Abbildung 5** fasst die Erkennung aller Antigene aus dem Patientenmodell D05-GS in so genannten Chrom-Freisetzungstests zusammen. Dabei wurden mit radioaktivem ⁵¹Cr chromierte B-Zellen des Patienten mit den angegebenen Peptiden, in Konzentrationen von 10³ nmol/l bis 10⁻⁴ nmol/l herunter titriert, beladen und mit peptidreaktiven T-Zellklonen für 4 Stunden koinkubiert. Erkennung der Peptide durch die T-Zellen resultierte in der Lyse der B-Zellen und damit der Freisetzung des Chroms aus den Zellen, welches aus dem Zellüberstand bestimmt wurde. (K im grauen Feld enthält Kontrollen: der geschlossene Kreis zeigt die Erkennung der B-Zellen ohne Peptid; das Dreieck zeigt die Erkennung autologer, chromierter Melanomzellen; E:T = Effektorzell-zu-Targetzell-Verhältnis.)

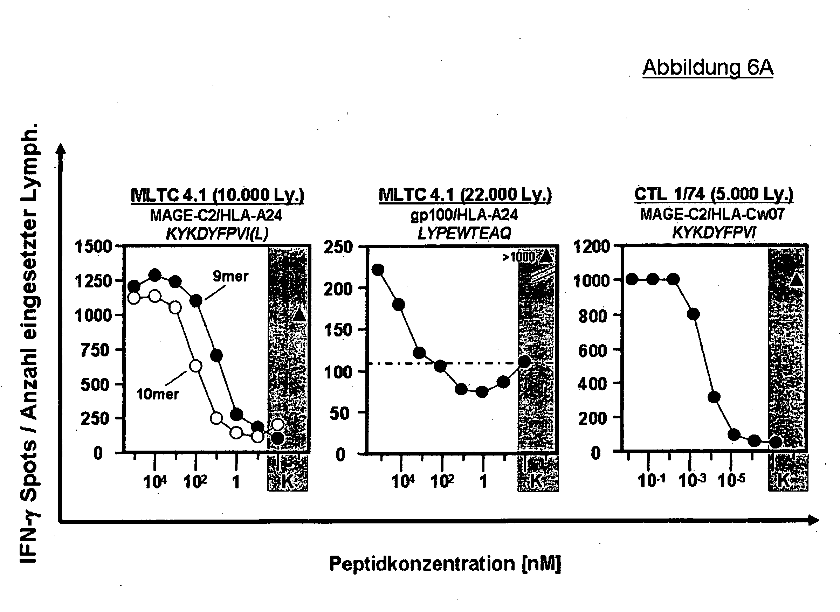

Die **Abbildungen 6A und 6B** fassen die Erkennung der Antigene aus dem Patientenmodell D14-SJR in IFN-γ ELISPOT-Assays zusammen. Auch hier dienten autologe B-Zellen oder mit einzelnen HLA-cDNAs transfizierte COS-7- oder 293 T-Zellen als Antigen präsentierende Zellen. Diese wurden mit den gezeigten Peptiden beladen und mit den T-Zellen für 20 Stunden koinkubiert. Auch in diesen Versuchen wurden die Peptide titriert. (K im grauen Feld in 6A enthält Kontrollen: der geschlossene Kreis zeigt die Erkennung der B-Zellen ohne Peptid, das Dreieck zeigt die Erkennung der D14-Melanomzellen.)

### Literatur:

T. Wölfel, A. Van Pel, E. De Plaen, C. Lurquin, J. Maryanski und T. Boon. Immunogenic (tum-) variants obtained by mutagenesis of mouse mastocytoma P815. VIII. Detection of stable transfectants expressing a tum- antigen with a cytolytic T cell stimulation assay. Immunogenetics 26: 178-187, 1987.
T. Wölfel, E. Klehmann, C. Müller, K.-H. Schütt, K.-H. Meyer zum Büschenfelde und A. Knuth. Lysis of human melanoma cells by autologous cytolytic T cell (CTL) clones: Identification of HLA-A2 as a restriction element for three different antigens. J. Exp. Med. 170 :797-810, 1989.
V. Brichard, A. Van Pel, T. Wölfel, C. Wölfel, E. de Plaen, B. Lethé, P. Coulie, und T. Boon. The tyrosinase gene codes for an antigen recognized by autologous cytolytic T lymphocytes on HLA-A2 melanomas. J. Exp. Med. 178: 489-495, 1993.
T. Wölfel, A. Van Pel, V. Brichard, J. Schneider, B. Seliger, K.-H. Meyer zum Büschenfelde, und T. Boon. Two tyrosinase nonapeptides recognized on HLA-A2 melanomas by autologous cytolytic T lymphocytes. Eur. J. Immunol. 24:759-764, 1994.
P. Coulie, V. Brichard, A. Van Pel, T. Wölfel, J. Schneider, C. Traversari, S. Mattei, E. De Plaen, C. Lurquin, J.-P. Szikora, J.-C. Renauld, und T. Boon. A new gene coding for a differentiation antigen recognized by autologous cytolytic T lymphocytes on HLA-A2 melanomas. J. Exp. Med. 180:35-42,1994.
T. Wölfel, M. Hauer, J. Schneider, M. Serrano, C. Wölfel, E. Klehmann-Hieb, E. De Plaen, T.Hankeln, K.-H. Meyer zum Büschenfelde, und D. Beach. A p16INK4a-insensitive CDK4 mutant targeted by cytolytic T lymphocytes in a human melanoma. Science 269:1281-1284, 1995.
V. G. Brichard, J. Herman, A. Van Pel, C. Wildman, B. Gaugler, T. Wölfel, T. Boon, und B. Lethé. A tyrosinase peptide presented by HLA-B44 is recognized by autologous cytolytic T lymphocytes. Eur. J. Immunol. 26:224-230, 1996.
W. Herr, J. Schneider, A.W. Lohse, K.-H. Meyer zum Büschenfelde, und T. Wölfel. Detection and quantification of blood-derived T lymphocytes secreting tumor necrosis factor alpha in response to HLA-A2.1-binding melanoma and viral peptide antigens. J.Immunol. Methods 191:131-142, 1996.
W. Herr, B. Linn, N. Leister, E. Wandel, K.-H. Meyer zum Büschenfelde, und T. Wölfel. The use of computer-assisted video image analysis for the quantification of CD8+ T lymphocytes producing tumor necrosis factor a spots in response to peptide antigens. J. Immunol. Methods 203:141-152, 1997.
J. Schneider, V. Brichard, T. Boon, K.-H. Meyer zum Büschenfelde, und T. Wölfel. Overlapping peptides of melanocyte differentiation antigen Melan-A/MART-1 recognized byautologous cytolytic T lymphocytes in association with HLA-B45.1 and HLA-A2.1. Int. J. Cancer 75:451-458, 1998.
S. Morel, A. Ooms, A. Van Pel, T. Wölfel, V. Brichard, P. van der Bruggen, B. Van den Eynde und G. Degiovanni. A new tyrosinase peptide presented by HLA-B35 is recognized on a human melanoma by autologous cytotoxic T lymphocytes. Int. J. Cancer 83:755-759, 1999.
C.Wölfel, LDrexler, A. Van Pel, T. Thres, N. Leister, W. Herr, G. Sutter, C. Huber und T. Wölfel. Transporter (TAP)- and proteasome-independent presentation of a melanomaassociated tyrosinase epitope. Int. J. Cancer 88:432-438, 2000.
C.M.Britten, R.G.Meyer, T.Kreer, I.Drexler, T.Wölfel, W.Herr. The use of HLA-A*0201-transfected K562 as standard antigen-presenting cells for CD8(+) T lymphocytes in IFN-gamma ELISPOT assays. J. Immunol. Methods 259:95-110, 2002.
R.Konopitzky, U.König, R.G.Meyer, W.Sommergruber, T.Wölfel, und T.Schweighoffer. Identification of HLA-A*0201-restricted T cell epitopes derived from the novel overexpressed tumor antigen CLCA2. J. Immunol., 169:540-547, 2002.
C.M.Britten, R.G.Meyer, N.Frankenberg, C.Huber. T.Wölfel. The use of clonal mRNA as antigenic format for the detection of antigen-specific T lymphocytes in IFN-gamma ELISPOT assays. J. Immunol. Methods, 287:125-136, 2004.
A.Dörrschuck, A.Schmidt, E. Schnürer, M. Glückmann, C.Albrecht, C.Wölfel, V.Lennerz, A.Lifke, C.Di Natale, E.Ranieri, L.Gesualdo, C.Huber, M.Karas, T.Wölfel, und W.Herr. CD8+ cytotoxic T lymphocytes isolated from allogeneic healthy donors recognize HLA-class Ia/Ib-associated renal carcinoma antigens with ubiquitous or restricted tissue expression. Blood, 104:2591-2599, 2004.
Y.Zhang. Z.Sun. H.Nicolay, R.G.Meyer, N.Renkvist, V.Stroobant, J.Corthals, J.Carrasco, A.M.Eggermont, M.Marchand, K.Thielemans, T.Wölfel, T.Boon, P.van der Bruggen. Monitoring of anti-vaccine CD4 T cell frequencies in melanoma patients vaccianted with a MAGE-3 protein. J. Immunol. 174:2404-2411, 2005.
R.G.Meyer, C.M.Britten, U.Siepmann, B.Petzold, T.A.Sagban, H.A.Lehr, B. Weigle, M.Schmitz, L.Mateo,B.Schmidt, H.Bernhard, T.Jakob, R.Hein, G.Schuler, B.Schuler-Thumer, S.N. Wagner, I.Drexler, G.Sutter, N.Arndtz, P.Chaplin, A.Enk, C.Huber, und T.Wölfel. A phase I vaccination study with tyrosinase in patients with stage II melanoma using recombinant modified vaccinia virus Ankara (MVA-hTyr). Cancer Immunol. Immunother. 54:453-467, 2005.
C.M.Britten, R.G.Meyer, C.Graf, C.Huber, T.Wölfel. Identification of T cell epitopes by the use of rapidly generated mRNA fragments. J. Immunol. Methods 299:165-175, 2005.

## Patentansprüche

1. Melanom-spezifisches Immunogen, das ein Peptide mit einer Länge von 10 bis 200 Resten ist und das die Aminosäurensequenz der SEQ ID Nr. 2 umfasst, wobei das Immunogen eine Melanom-spezifische HLA-restringierte CTL-Antwort hervorruft.

2. Immunogen nach Anspruch 1, wobei das Immunogen eine Länge von 10 bis 11 Resten aufweist.

3. Immunogen nach Anspruch 1, wobei das Immunogen mit dem Peptid der SEQ ID Nr. 2 identisch ist.

4. Immunogen nach einem der Ansprüche 1 bis 3, wobei das Immunogen eine durch Aminosäure-Substitution, -Deletion oder -Insertion einer einzigen Aminosäure davon ableitbare Aminosäuresequenz aufweist, die ein funktionelles Äquivalent zu der Aminosäuresequenz zu einem Peptid der SEQ ID Nr. 2 darstellt, und es ein Epitop für CD8-positive CTL darstellt und dazu geeignet ist, eine auf humanes Leukozyten-Antigen der Molekülgruppe MHC Klasse I, Allelvariante A oder B restringierte Immunantwort von CD8-positiven CTL gegen Tumorzellen zu induzieren.

5. Retro-inverses Peptid oder Pseudopeptid, **dadurch gekennzeichnet, dass** es einem Immunogen einem der Ansprüche 1 bis 4 entspricht, bei dem anstelle der -CO-NH-Peptidbindungen - NH-CO-Bindungen ausgebildet sind.

6. Polynukleotid, umfassend eine Nukleotidsequenz, die für ein Immunogen nach einem der Ansprüche 1 bis 4 kodiert.

7. Pharmazeutische Zusammensetzung zur *in vivo* oder *in vitro* Aktivierung von T-Zellen, insbesondere CD8-positiven cytotoxischen T-Lymphozyten, **dadurch gekennzeichnet, dass** die Zusammensetzung mindestens ein Immunogen nach einem der Ansprüche 1 bis 4 und/oder mindestens ein retro-inverses Peptids oder Pseudopeptids nach Anspruch 5 und/oder eines Polynukleotid nach Anspruch 6 umfaßt, gegebenenfalls mit entsprechenden akzeptablen Trägern und Hilfsstoffen.

8. Rekombinantes DNA- oder RNA-Vektormolekül, das mindestens ein oder mehrere Polynukleotid(e) nach Anspruch 6 enthält und das in Zellen autologen, allogenen, xenogenen oder mikrobiologischen Ursprungs exprimierbar ist.

9. Wirtszelle, die ein Polynukleotid gemäß Anspruch 6 oder ein Vektormolekül gemäß Anspruch 8 enthält.

10. Verwendung mindestens eines Immunogens nach einem der Ansprüche 1 bis 4 und/öder eines retro-inversen Peptids oder Pseudopeptids gemäß Anspruch 5 zur Herstellung polyklonaler, monoklonaler oder rekombinanter Antikörper gegen das/die betreffende(n) Immunogen(e) oder gegen einen Komplex aus dem/den betreffenden Immunogen(en) und HLA.

11. Antikörper, der spezifisch mit wenigstens einem Immunogen nach einem der Ansprüche 1 bis 4 und/oder einem retro-inversen Peptid oder Pseudopeptid gemäß Anspruch 5 oder mit einem Komplex aus dem/den betreffenden Immunogen(en) und HLA reagiert.

12. Verwendung mindestens eines Immunogens nach einem der Ansprüche 1 bis 4 und/oder eines retro-inversen Peptids oder Pseudopeptids gemäß Anspruch 5 zur Herstellung polyklonaler oder monoklonaler oder rekombinanter T-Zellrezeptoren oder dazu funktionell äquivalenter Moleküle gegen das/die betreffenden(n) Immunogen(e).

13. T-Zellrezeptor oder dazu funktionell äquivalentes Molekül, der/das spezifisch mit wenigstens einem Immunogen nach einem der Ansprüche 1 bis 4 und/oder einem retro-inversen Peptid oder Pseudopeptid nach Anspruch 5 reagiert.

14. Polynukleotid, das für einen T-Zellrezeptor nach Anspruch 13 kodiert.

15. Expressionsvektor, der einen T-Zellrezeptor nach Anspruch 13 exprimiert.

16. Wirtszelle, die ein Polynukleotid nach Anspruch 14 oder einen Expressionsvektor nach Anspruch 15 enthält.

17. Arzneimittel zur Behandlung von Erkrankungen, die mit einem der Immunogene nach einem der Ansprüche 1 bis 4 assoziiert sind, **dadurch gekennzeichnet, dass** das Arzneimittel mindestens ein Immunogen und/oder mindestens ein retro-inverses Peptid oder Pseudopeptid und/oder mindestens ein Polynukleotid und/oder mindestens einen T-Zellrezeptor und/oder mindestens ein Vektormolekül und/oder mindestens eine Wirtszelle und/oder mindestens ein Antikörper nach einem der vorgenannten Ansprüche, gegebenenfalls zusammen mit geeigneten Zusatz- und Hilfsstoffen, enthält.

18. Verwendung eines Immunogens nach einem der Ansprüche 1 bis 4 und/oder eines retro-inversen Peptids oder Pseudopeptids gemäß Anspruch 5 zur Herstellung von Diagnostika und/oder Therapeutika und/oder Prophylaktika für den Nachweis und/oder die Beeinflussung und/oder Generierung und/oder Expandierung und/oder Steuerung des Aktivierungs- und Funktionszustands von T-Zellen, insbesondere CD8-positiven cytotoxischen T-Lymphozyten.

## Claims

1. Melanoma specific immunogen, which is a peptide having a length of 10 to 200 residues and comprises the amino acid sequence of SEQ ID No. 2, wherein said immunogen elicits a melanoma-specific HLA-restringed CTL-response.

2. Immunogen according to claim 1, wherein said immunogen has a length of 10 to 11 residues.

3. Immunogen according to claim 1, wherein said immunogen is identical to the peptide of SEQ ID No. 2.

4. Immunogen according to any of claims 1 to 3, wherein said immunogen has an amino acid sequence which can be derived by an amino acid substitution, deletion or insertion of a single amino acid providing a functional equivalent to the amino acid sequence of a peptide of SEQ ID No. 2, and wherein said immunogen provides an epitope for CD8-positive CTL and is suitable to induce an immune response of CD8-positive CTL restringed to human leukocyte antigen from the group of molecules MHC class I, allelic variant A or B, against tumor cells.

5. Retro-inverse peptide or pseudopeptide, **characterized in that** it corresponds to an immunogen according to any of claims 1 to 4, wherein NH-CO-bonds are formed in place of the -CO-NH-peptide bonds.

6. Polynucleotide, comprising a nucleotide sequence encoding for an immunogen according to any of claims 1 to 4.

7. Pharmaceutical composition for an *in vivo* or *in vitro* killing of T-cells, in particular CD8-positive cytotoxic T-lymphocytes, **characterized in that** said composition comprises at least one immunogen according to any of claims 1 to 4 and/or at least one retro-inverse peptide or pseudopeptide according to claim 5 and/or a polynucleotide according to claim 6, optionally with respective acceptable carriers and auxiliary agents.

8. Recombinant DNA or RNA vector molecule, containing at least one or more polynucleotide(s) according to claim 6 which can be expressed in cells of autologous, allogenic, xenogenic or microbiological origin.

9. Host cell, containing a polynucleotide according to claim 6 or a vector molecule according to claim 8.

10. Use of at least one immunogen according to any of claims 1 to 4 and/or a retro-inverse peptide or pseudopeptide according to claim 5 for producing polyclonal, monoclonal or recombinant antibodies against the respective immunogen(s) or against a complex of the respective immunogen(s) and HLA.

11. Antibody that specifically reacts with at least one immunogen according to any of claims 1 to 4 and/or a retro-inverse peptide or pseudopeptide according to claim 5 or with a complex of the respective immunogen(s) and HLA.

12. Use of at least one immunogen according to any of claims 1 to 4 and/or a retro-inverse peptide or pseudopeptide according to claim 5 for producing polyclonal or monoclonal or recombinant T-cell receptors or molecules that are functionally equivalent thereto against the respective immunogen(s).

13. T-cell receptor or molecule that is functionally equivalent thereto which specifically reacts with at least one immunogen according to any of claims 1 to 4 and/or a retro-inverse peptide or pseudopeptide according to claim 5.

14. Polynucleotide, encoding for a T-cell receptor according to claim 13.

15. Expression vector, expressing a T-cell receptor according to claim 13.

16. Host cell containing a polynucleotide according to claim 14 or an expression vector according to claim 15.

17. Medicament for the treatment of diseases that are associated with one of the immunogens according to any of claims 1 to 4, **characterized in that** said medicament contains at least one immunogen and/or at least one retro-inverse peptide or pseudopeptide and/or at least one polynucleotide and/or at least one T-cell receptor and/or at least one vector molecule and/or at least one host cell and/or at least one antibody according to any of the preceding claims, optionally together with suitable additives and auxiliary agents.

18. Use of an immunogen according to any of claims 1 to 4 and/or a retro-inverse peptide or pseudopeptide according to claim 5 for producing diagnostics and/or therapeutics and/or prophylactics for the detection and/or the manipulation and/or generation and/or expansion and/or control of the state of the activity and function of T-cells, in particular of CD8-positive cytotoxic T-lymphocytes.

## Revendications

1. Immunogène spécifique à un mélanome, qui est un peptide d'une longueur de 10 à 200 résidus et qui comprend la séquence d'acides aminés de SEQ ID N°2, l'immunogène provoquant une réponse CTL restreinte par le HLA spécifique à un mélanome.

2. Immunogène selon la revendication 1, dans lequel l'immunogène présente une longueur de 10 à 11 résidus.

3. Immunogène selon la revendication 1, dans lequel l'immunogène est identique au peptide de SEQ ID N°2.

4. Immunogène selon l'une des revendications 1 à 3, dans lequel l'immunogène présente une séquence d'acides aminés pouvant être dérivée par substitution, délétion ou insertion d'un unique acide aminé de celle-ci, qui représente un équivalent fonctionnel de la séquence d'acides aminés d'un peptide de SEQ ID N°2, et représente un épitope pour les CTL CD8-positifs et est approprié à induire une réponse immunitaire des CTL C8-positifs contre des cellules tumorales, restreinte à l'antigène des leucocytes humains du groupe de molécules CHM de catégorie I, variante allélique A ou B.

5. Peptide ou pseudopeptide rétro-inversé, **caractérisé en ce qu'**il correspond à un immunogène selon l'une des revendications 1 à 4, dans lequel des liaisons -NH-CO sont formées à la place des liaisons peptidiques -CO-NH.

6. Polynucléotide, comprenant une séquence nucléotidique qui code un immunogène selon l'une des revendications 1 à 4.

7. Composition pharmaceutique pour l'activation *in vivo* ou *in vitro* de cellules T, en particulier de lymphocytes T cytotoxiques CD8-positifs, **caractérisée en ce que** la composition comprend au moins un immunogène selon l'une des revendications 1 à 4 et/ou au moins un peptide ou pseudopeptide rétro-inversé selon la revendication 5 et/ou un polynucléotide selon la revendication 6, le cas échéant avec des excipients et auxiliaires pharmaceutiquement acceptables.

8. Molécule vecteur d'ADN ou d'ARN recombinante, qui comprend au moins un ou plusieurs polynucléotide(s) selon la revendication 6 et qui peut être exprimée par dans des cellules d'origine autologue, allogène, xénogène ou microbiologique.

9. Cellule hôte, qui comprend un polynucléotide selon la revendication 6 ou une molécule vecteur selon la revendication 8.

10. Utilisation d'au moins un immunogène selon l'une des revendications 1 à 4 et/ou d'un peptide ou pseudopeptide rétro-inversé selon la revendication 5 pour la préparation d'anticorps polyclonaux, monoclonaux ou recombinants contre le ou les immunogènes concernés ou contre un complexe constitué du ou des immunogènes concernés et du HLA.

11. Anticorps qui réagit spécifiquement avec au moins un immunogène selon l'une des revendications 1 à 4 et/ou un peptide ou pseudopeptide rétro-inversé selon la revendication 5 ou avec un complexe constitué du ou des immunogènes concernés et du HLA.

12. Utilisation d'au moins un immunogène selon l'une des revendications 1 à 4 et/ou d'un peptide ou pseudopeptide rétro-inversé selon la revendication 5 pour la préparation de récepteurs de cellules T polyclonaux ou monoclonaux ou recombinants ou de molécules fonctionnellement équivalentes à ceux-ci dirigés contre le ou les immunogènes concernés.

13. Récepteur des cellules T ou molécule fonctionnellement équivalente à celui-ci, qui réagit spécifiquement avec un immunogène selon l'une des revendications 1 à 4 et/ou un peptide ou pseudopeptide rétro-inversé selon la revendication 5.

14. Polynucléotide qui code un récepteur de cellules T selon la revendication 13.

15. Vecteur d'expression qui exprime un récepteur de cellules T selon la revendication 13.

16. Cellule hôte qui contient un polynucléotide selon la revendication 14 ou un vecteur d'expression selon la revendication 15.

17. Médicament destiné au traitement de maladies qui sont associées à un des immunogènes selon l'une des revendications 1 à 4, **caractérisé en ce que** le médicament contient au moins un immunogène et/ou au moins un peptide ou pseudopeptide rétro-inversé et/ou au moins un polynucléotide et/ou au moins un récepteur des cellules T et/ou au moins une molécule vecteur et/ou au moins une cellule hôte et/ou au moins un anticorps selon l'une des revendications précédentes, le cas échéant conjointement avec des additifs et auxiliaires appropriés.

18. Utilisation d'un immunogène selon l'une des revendications 1 à 4 et/ou d'un peptide ou pseudopeptide rétro-inversé selon la revendication 5 pour la préparation de produits diagnostiques et/ou thérapeutiques et/ou prophylactiques pour la détection et/ou l'influence et/ou la génération et/ou l'extension et/ou le contrôle de l'état d'activation et de fonction des cellules T, en particulier des lymphocytes T cytotoxiques CD8-positifs.
